# EUROPÄISCHE PATENTSCHRIFT

(11) **EP 3 229 763 B1**
(45) Veröffentlichungstag und Bekanntmachung des Hinweises auf die Patenterteilung: **18.09.2019**
(21) Anmeldenummer: 15787628.5
(22) Anmeldetag: 02.11.2015
(51) Int. Cl.: A61K 8/34, A61K 8/41, A61Q 5/00, A61K 8/64, A61Q 5/12, A61K 8/891, A61K 8/894, A61K 8/898, A61K 8/04

(54) **MITTEL UND VERFAHREN ZUR PFLEGE KERATINHALTIGER FASERN**
KERATINOUS FIBER CARE PRODUCT AND METHOD
PRODUIT ET PROCÉDÉS POUR SOINS DE FIBRES KÉRATINIQUES

(30) Priorität: 10.12.2014 DE 102014225425
(43) Veröffentlichungstag der Anmeldung: 18.10.2017
(73) Patentinhaber: Henkel AG & Co. KGaA, 40589 Düsseldorf (DE)
(72) Erfinder: SCHEUNEMANN, Volker, 21339 Lüneburg (DE); KNAPPE, Thorsten, 22869 Schenefeld (DE); SCHULZE ZUR WIESCHE, Erik, 22453 Hamburg (DE)
(86) Internationale Anmeldenummer: PCT/EP2015/075397
(87) Internationale Veröffentlichungsnummer: WO 2016/091472

(56) Entgegenhaltungen:
- EP-A1- 0 686 388
- WO-A2-02/12090
- DE-A1- 19 907 715
- DE-U1-202005 009 617
- JP-A- 2007 319 234
- US-A- 3 372 840
- US-A- 4 067 480
- US-A- 5 885 561
- US-A1- 2002 074 349
- US-A1- 2002 079 377
- US-A1- 2004 065 683
- US-A1- 2009 061 004
- US-A1- 2009 098 079
- US-A1- 2013 018 333

## Beschreibung

Die Anmeldung betrifft das technische Fachgebiet der Pflege keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur temporären Umformung keratinhaltiger Fasern Gegenstand der vorliegenden Anmeldung.

Eine ansprechend aussehende Frisur wird heute allgemein als unverzichtbarer Teil eines gepflegten Äußeren angesehen. Um eine solche ansprechende Frisur zu realisieren werden die Haare kosmetischen Behandlungsverfahren unterworfen, die von der Reinigung mittels eines Shampoos bis zur permanenten Verformung mittels chemisch/thermischer Verfahren oder der permanenten oxidativen Färbung reichen. Viele dieser kosmetischen Verfahren sind mit einem Eingriff in die Haarstruktur verbunden und je nach Häufigkeit und Intensität der Behandlung sind diese Verfahren geeignet, das Haar zu schädigen.

Zur Vorbeugung einer solchen Schädigung oder zur Beseitigung bereits entstandener Schädigungen stehen Haarpflegemittel zur Verfügung. Bei der Anwendung dieser Haarpflegemittel hat insbesondere die Sprühapplikation entsprechender kosmetischer Zubereitungen eine große Bedeutung, wobei die Zubereitungen in der Regel als Pumpsprays oder Aerosolsprays appliziert werden. Hierzu werden die kosmetischen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen, wie z.B. in der DE202005009617 beschrieben. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel. Aufgabe der vorliegenden Erfindung war es daher, spezifische haarkosmetische Zubereitungen zur Pflege keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens eine hohe Pflegewirkung, sowie vorzugsweise eine Haltewirkung, insbesondere einen hohen Langzeithaltegrad, und einen hohen Volumeneffekt realisieren. Es hat sich gezeigt, dass zur Lösung dieser Aufgabe aus der Vielzahl bekannten haarkosmetisch wirksamen Zubereitungen insbesondere Zubereitungen mit hohem Lösungsmittelanteil auf Grundlage pflegender Komponenten geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), wie definiert unter b) in Anspruch 1.

Die kosmetische Zubereitung a) ist flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Die erfindungsgemäße kosmetische Zubereitung enthält als ersten wesentlichen Bestandteil mindestens 88 Gew.-% eines polaren Lösungsmittels a1). Bevorzugte kosmetische Produkte sind dadurch gekennzeichnet, dass dass der Gewichtsanteil des polaren Lösungsmittels a1) am Gesamtgewicht der kosmetischen Zubereitung a) 80 bis 99 Gew.-%, vorzugsweise 90 bis 98 Gew.-% und insbesondere 92 bis 97 Gew.-% beträgt. Entsprechende Mittel zeichnen sich durch eine gute kosmetische Wirkung bei gleichzeitig guter Applizierbarkeit aus.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol und Wasser erwiesen, welche aus diesem Grund als polare Lösungsmittel a1) bevorzugt werden.

Besonders bevorzugte polare Lösungsmittel a1) oder Lösungsmittelsysteme sind
- polare Lösungsmittel a1), bei denen der Gewichtsanteil von Wasser am Gesamtgewicht des polaren Lösungsmittels a1) mehr als 80 Gew.-%, vorzugsweise mehr als 85 Gew.-% und insbesondere mehr als 90 Gew.-% beträgt;
- polare Lösungsmittel a1), bei der Gewichtsanteil von Wasser am Gesamtgewicht des polaren Lösungsmittels a1) mehr als 80 Gew.-%, vorzugsweise mehr als 85 Gew.-% und insbesondere mehr als 90 Gew.-% beträgt.
- polare Lösungsmittel a1), bei denen das polare Lösungsmittel a1) Wasser und Ethanol umfasst und Gewichtsverhältnis von Wasser zu Ethanol 50:1 bis 1:1, bevorzugt 40:1 bis 2:1 und insbesondere 25:1 bis 5:1 beträgt.

Ein zweiter wesentlicher Bestandteil erfindungsgemäßer kosmetischer Zusammensetzungen ist die Pflegekomponente a2). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil der Pflegekomponente am Gesamtgewicht der kosmetischen Zubereitung 0,2 bis 18 Gew.-%, vorzugsweise 0,5 bis 12 Gew.-% und insbesondere 1,0 bis 10 Gew.-% beträgt.

Besonders bevorzugte Pflegekomponenten sind ausgewählt aus der Gruppe der
i) kationischen Polymere;
ii) kationischen Tenside;
iii) Silikone, insbesondere aus der Gruppe der
   - alkoxylierten Dimethicone, vorzugsweise aus der Gruppe der ethoxylierten Dimethicone und insbesondere aus der Gruppe der Verbindungen mit der INCI-Bezeichnung PEG-12 Dimethicone;
   - aminofunktionellen Silikone;
iv) Polyole;
v) Proteinhydrolysate, insbesondere aus der Gruppe der Gruppe der Hydrolysate von Keratin, Seidenprotein und Weizenprotein;
vi) Wachse.

Die kosmetische Zubereitung a) kann eine oder mehr verschiedene Pflegekomponenten enthalten. Bevorzugt werden kosmetische Produkte, bei denen die kosmetische Zubereitung mindestens zwei, vorzugsweise mindestens drei und insbesondere mindestens vier voneinander verschiedene Pflegekomponenten a2) umfasst.

Einige besonders bevorzugte Pflegestoffkombinationen sind in den nachfolgenden Tabellen dargestellt:

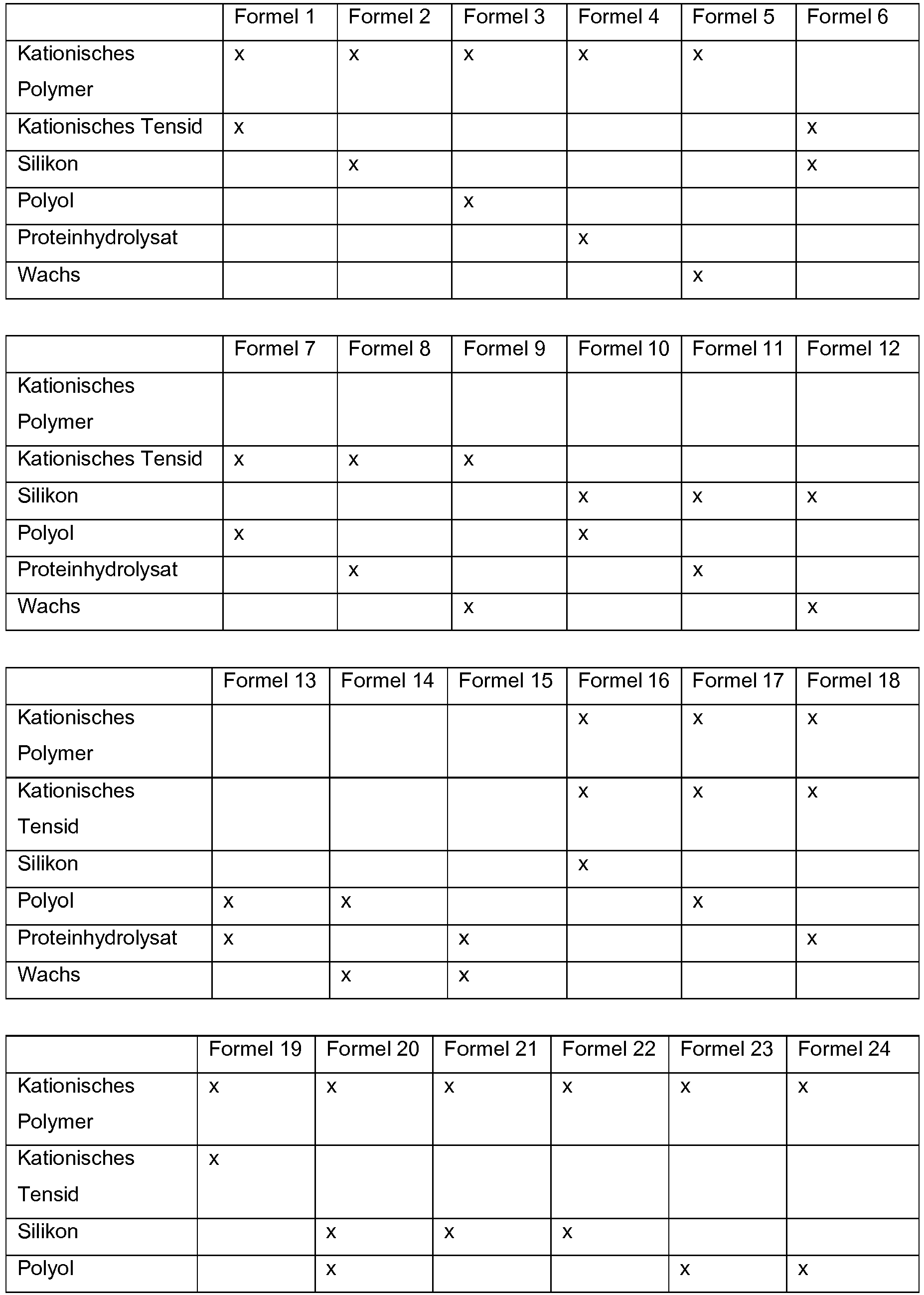

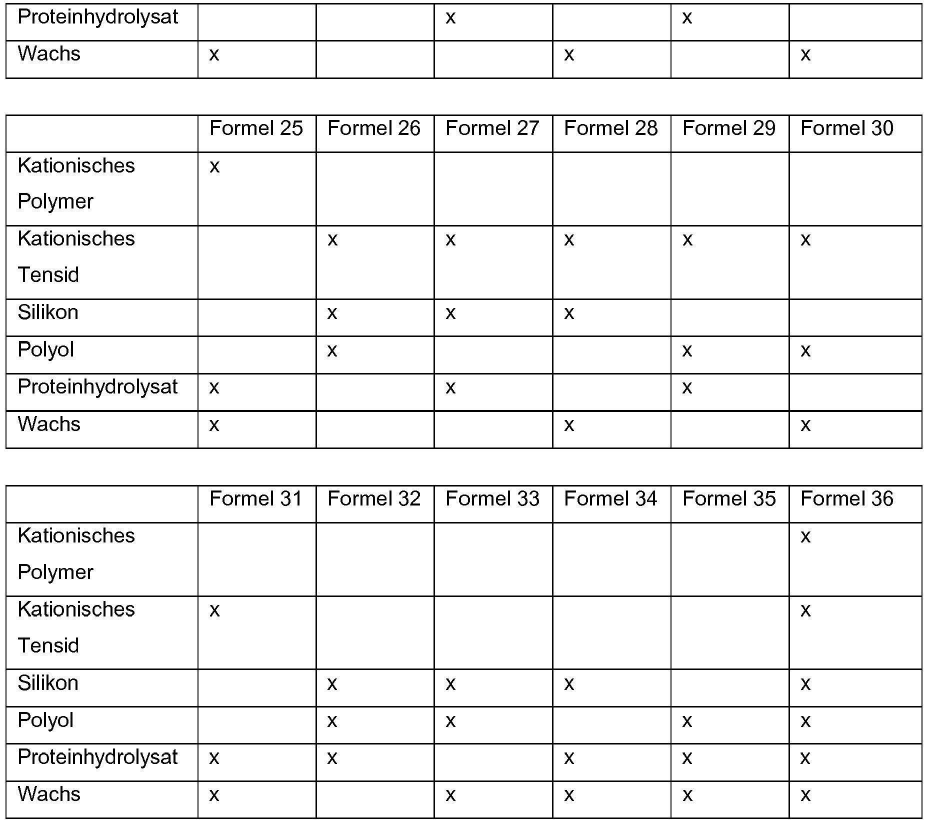

Eine erste Gruppe besonders bevorzugter Pflegekomponenten a2) bilden die kationischen Polymere.

Die Gruppe der kationischen Polymere a3) umfasst insbesondere die kationische Polymere mit den INCI Bezeichnungen Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polyquaternium-67, Polyquaternium-68 und Polyquaternium-69.

Als kationisches Polymere werden insbesondere die kationischen Cellulosen bevorzugt. Bevorzugte kosmetisches Produkte sind dadurch gekennzeichnet, dass die Pflegekomponte a2) ausgewählt ist aus der Gruppe der kationischen Cellulosederivate. Beispiele für kationische Cellulosen sind die Polymere mit den Handelsnamen Celquat H 100 und Celquat L 200 (Polyquaternium-4), Polymer JR 400 (INCI-Bezeichnung Polyquaternium-10), Polymer LM-200 (INCI-Bezeichnung Polyquaternium-24), sowie Mirustyle CP (Polyquaternium-72). Geeignete kationische Guarderivate werden beispielsweise unter den Handelsbezeichnungen Jaguar, N-Hance, Cosmedia Guar und AquaCat (INCI-Bezeichnung Guar Hydroxypropyltrimonium Chloride) vertrieben.

Erfindungsgemäß besonders geeignete Komponenten kationische Polymere sind quaternisierte Hydroxyethylcellulosepolymere, die mit kationischen Trimethylammonium- und Dimethyldodecylammoniumgruppen substituiert wurden. Die kationische Substituierung der Polymere bewirkt eine gesteigerte Substantivität der Polymere auf den Haaren, denn die Oberfläche der Haare ist negativ geladen, während die hydrophobe Modifizierung (die Dodecylammoniumgruppe) die Abscheidung weiterer konditionierender Stoffe aus dem Haarkonditioniermittel auf der Kopfhaut und dem Haar unterstützt.

Erfindungsgemäß ganz besonders bevorzugte kationische Polymere weisen einen kationischen Substitutionsgrad von 0,15 bis 0,25 und einen hydrophoben Substitutionsgrad (HS) von HS ≤ 0,01 auf. Durchschnittlich enthalten diese kationischen Polymere einen prozentualen Stickstoffgehalt im Molekül von 0,5 bis 3%, bevorzugt von 0,6 bis 2,7% und insbesondere von 0,8 bis 2,4%. Solche Polymere sind bekannt unter der INCI-Bezeichnung Polyquaternium-67 und werden im Handel beispielsweise von der Firma Amerchol unter den Bezeichnungen Polymer® SL oder Polymer® SK angeboten.

Bevorzugte kosmetische Zubereitungen a) enthalten bezogen auf ihr Gesamtgewicht 0,01 bis 10 Gew.-%, vorzugsweise 0,05 bis 5,0 Gew.-% und insbesondere 0,1 bis 3,0 Gew.-% kationisches Poylmer.

Eine zweite Gruppe bevorzugter Pflegekomponenten a2) bilden die kationischen Tenside, insbesondere die kationischen Tenside aus der Gruppe der quartären Ammoniumverbindungen, der Esterquats und der Amidoamine

Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Behenyltrimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Ganz besonders bevorzugte erfindungsgemäße kosmetische Zubereitungen a) sind dadurch gekennzeichnet, dass sie (C₁₂ bis C₁₈)-Alkyltrimethylammoniumsalz(e) enthalten.

Besonders bevorzugt sind Cetyltrimethylammonium- und Behenyltrimethylammoniumsalze. Ganz besonders bevorzugt sind Cetyltrimethylammoniumsalze und Behenyltrimethylammoniumsalze in Form der Methosulfate und/oder der Chloride.

Als erfindungsgemäß zu verwendende Amidoamine, welche gegebenenfalls quaternisiert sein können, kommen beispielsweise in Betracht: Tego Amid® S18 (Evonik; INCI-Bezeichnung: Stearamidopropyl Dimethylamine), Lexamine® S 13 (Inolex; INCI-Bezeichnung: Stearamidopropyl Dimethylamine), Incromine® SB (Croda; INCI-Bezeichnung: Stearamidopropyl Dimethylamine), Witcamine 100 (Witco, INCI-Bezeichnung: Cocamidopropyl Dimethylamine), Incromine BB (Croda, INCI-Bezeichnung: Behenamidopropyl Dimethylamine), Mackine 401 (Mclntyre, INCI-Bezeichnung: Isostearylamidopropyl Dimethylamine) und andere Mackine-Typen, Adogen S18V (Witco, INCI-Bezeichnung: Stearylamidopropyl Dimethylamine), und als permanent kationische Aminoamine: Rewoquat RTM 50 (Witco Surfactants GmbH, INCI-Bezeichnung: Ricinoleamidopropyltrimonium Methosulfate), Empigen CSC (Albright&Wilson, INCI-Bezeichnung: Cocamidopropyltrimonium Chlorid), Swanol Lanoquat DES-50 (Nikko, INCI-Bezeichnung: Quatemium-33), Rewoquat UTM 50 (Witco Surfactants GmbH, Undecyleneamidopropyltrimonium Methosulfate). Besonders bevorzugt ist Stearamidopropyl Dimethylamine.

Bevorzugte kosmetische Zubereitungen a) enthalten bezogen auf ihr Gesamtgewicht 0,01 bis 10 Gew.-%, vorzugsweise 0,05 bis 5,0 Gew.-% und insbesondere 0,1 bis 3,0 Gew.-% kationisches Tensid.

Eine dritte Gruppe bevorzugter Pflegekomponenten a2) bilden die Silikone, insbesondere die
- alkoxylierten Dimethicone, vorzugsweise aus der Gruppe der ethoxylierten/propoxylierten Dimethicone;
- aminofunktionellen Silikone.

Zur Gruppe der alkoxylierten Dimethicone zählen beispielsweise
- ethoxylierten Dimethicone mit der INCI-Bezeichnung PEG-x Dimethicone mit x = 2 bis 20, vorzugsweise 3 bis 17 und insbesondere 11 oder 12;
- der ethoxylierten Dimethicone mit der INCI-Bezeichnung Bis-PEG-y Dimethicone mit x= 3 bis 25, vorzugsweise 4 bis 20;
- der ethoxylierten/propoxylierten Dimethicone mit der INCI-Bezeichnung PEG/PPG a/b Dimethicone, wobei a und b unabhängig voneinander für Zahlen von 2 bis 30, vorzugsweise von 12 bis 24 und insbesondere von 14 bis 20 stehen;
- der ethoxylierten/propoxylierten Dimethicone mit der INCI-Bezeichnung Bis-PEG/PPG-c/d Dimethicone, wobei c und d unabhängig voneinander für Zahlen von 10 bis 25, bevorzugt 14 bis 20 und insbesondere von 14 bis 16 stehen
- der ethoxylierten/propoxylierten Dimethicone mit der INCI-Bezeichnung Bis-PEG/PPG-e/f PEG/PPG g/h Dimethicone, wobei e, f, g und h unabhängig voneinander für Zahlen von 10 bis 20, vorzugsweise 14 bis 18 und insbesondere 16 stehen.

Die Gruppe der aminofunktionellen Silikone, welche mindestens eine, gegebenenfalls substituierte Aminogruppe aufweisen, werden nach der INCI-Deklaration als Amodimethicone bezeichnet.

Bevorzugte kosmetische Zubereitungen enthalten ein oder mehrere aminofunktionelle Silikone der Formel M(RₐQ_{b}SiO_{(4-a-b)/2)x}(R_{c}SiO_{(4-c)/2)y}M (Si-1) beschrieben werden, wobei in der obigen Formel
R ein Kohlenwasserstoff oder ein Kohlenwasserstoffrest mit 1 bis etwa 6 Kohlenstoffatomen, vorzugsweise Methyl ist,
Q ein polarer Rest der allgemeinen Formel -R¹HZ, vorzugsweise ein Rest der Formel - CH₂CH₂CH₂NHCH₂CH₂NH₂ ist,
   worin
R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
Z ein organischer, aminofunktioneller Rest ist, der mindestens eine aminofunktionelle Gruppe enthält, wobei es sich vorzugsweise um einen -NHCH₂CH₂NH₂-Rest oder einen Rest der Formel -CH₂CH₂CH₂NHCH₂CH₂NH₂ handelt;
a Werte im Bereich von etwa 0 bis etwa 2 annimmt,
b Werte im Bereich von etwa 1 bis etwa 3 annimmt,
a + b kleiner als oder gleich 3 ist, und
c eine Zahl im Bereich von etwa 1 bis etwa 3 ist, und
x eine Zahl im Bereich von 1 bis etwa 2.000, vorzugsweise von etwa 3 bis etwa 50 und am bevorzugtesten von etwa 3 bis etwa 25 ist, und
y eine Zahl im Bereich von etwa 20 bis etwa 10.000, vorzugsweise von etwa 125 bis etwa 10.000 und am bevorzugtesten von etwa 150 bis etwa 1.000 ist, und
M eine geeignete Silikon-Endgruppe ist, wie sie im Stande der Technik bekannt ist, vorzugsweise Trimethylsiloxy.

Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und - (CH₂)₃C(O)SCH₂CH₂- ein.

Bevorzugte erfindungsgemäße kosmetische Zubereitungen enthalten ein aminofunktionelles Silikon der Formel R'ₐG₃₋ₐ-Si(OSiG₂)ₙ-(OSiG_{b}R'_{2-b})ₘ-O-SiG₃₋ₐ-R'ₐ (Si-2), worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus

-Q-N(R")-CH₂-CH₂-N(R")₂

-Q-N(R")₂

-Q-N⁺(R")₃A⁻

-Q-N⁺H(R")₂A⁻

-Q-WH₂(R")A⁻

-Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, lodid oder Methosulfat.

Besonders bevorzugte kosmetische Zubereitungen sind dadurch gekennzeichnet, dass sie mindestens es ein aminofunktionelles Silikon der Formel (Si2-a) 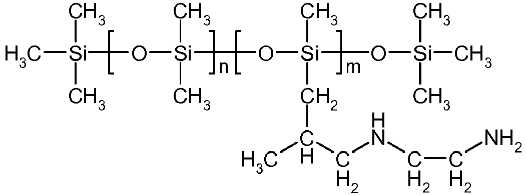 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

In einer weiteren bevorzugten Ausführungsform enthalten kosmetische Zubereitungen ein aminofunktionelles Silikon der Formel (Si-2b) 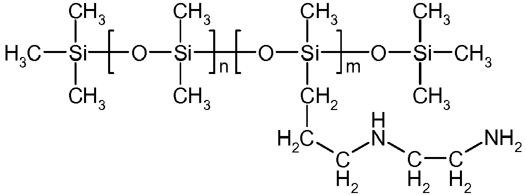 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone der Formel (Si2-a) und (Si2-b) werden nach der INCI-Deklaration als Amodimethicone bezeichnet. Entsprechende Amodimethicone sind beispielsweise in Form einer Emulsion als Handelsprodukt Dow Corning 939 oder als Handelsprodukt Dow Corning 949 erhältlich.

In einer alternativen Ausführungsform handelt es sich bei dem aminofunktionellen Silikon a2) jedoch um ein funktionalisiertes Amodimethicon. Entsprechende funktionalisierte Amodimethicone werden beispielsweise durch die Formel (Si-2c) 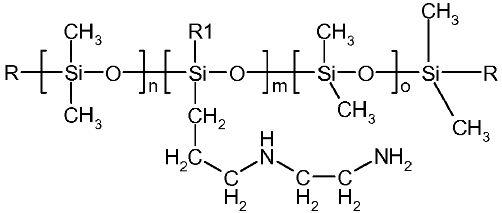 beschrieben, worin
R für -OH, eine (gegebenenfalls ethoxylierte und/oder propoxylierte) (C₁ bis C₂₀)-Alkoxygruppe oder eine -CH₃-Gruppe steht,
R1 für -OH, eine (C₁ bis C₂₀)-Alkoxygruppe oder eine -CH₃-Gruppe und
m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Bevorzugt werden beispielsweise aminofunktionelle Silikon a2) aus der Gruppe der hydroxylaminomodifizierten Silikone, bei denen in der obigen Formel (Si-2b) der Rest R für -OH steht. Entsprechende Silikone mit der INCI Bezeichnung Amodimethicone sind beispielsweise als Handelsprodukt Wacker Belsil ADM 652 erhältlich.

Steht der Rest R für eine (gegebenenfalls ethoxylierte und/oder propoxylierte) (C₁ bis C₂₀)-Alkoxygruppe, so fallen unter diese allgemeine Formel (Si-2b) beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich).

Unabhängig davon, welche aminofunktionellen Silikone eingesetzt werden, sind erfindungsgemäße kosmetische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Bevorzugte kosmetische Zubereitungen a) enthalten bezogen auf ihr Gesamtgewicht 0,01 bis 30 Gew.-%, vorzugsweise 0,05 bis 20 Gew.-% und insbesondere 0,1 bis 5,0 Gew.-% Silikon.

Eine vierte Gruppe bevorzugter Pflegestoffe a2) bilden die Polyole. Das Polyol ist von dem polaren Lösungsmittel a1) verschieden. Als Polyole eignen sich dabei insbesondere Polyole aus der Gruppe Glycerin, 1,2-Ethandiol, Polyethylenglycole mit MW > 400, Propandiol, Butandiol, inbesondere 1,3 Butandiol, Hexandiol, insbesondere 1,6 Hexandiol, Sorbitol, Threitol, Erythritol, Arabitol, Altritol, Ribitol, Xylitol, Galactitol, Mannitol, Iditol und Panthenol. Besonders geeignete Polyole sind Glycerin, Sorbitol und Panthenol.

Das Polyol kann als Einzelsubstanz oder in Form von Polyol Mischungen eingesetzt werden. Bevorzugte erfindungsgemäße Mittel zeichnen sich dadurch aus, dass sie weniger als vier, vorzugsweise ein bis drei Polyole enthalten.

Bevorzugte kosmetische Zubereitungen a) enthalten bezogen auf ihr Gesamtgewicht 0,01 bis 20 Gew.-%, vorzugsweise 0,05 bis 10 Gew.-% und insbesondere 0,1 bis 5,0 Gew.-% Polyol.

Die fünfte Gruppe bevorzugter Pflegestoffe a2) bilden die Proteinhydrolysate. Die Gruppe der besonders bevorzugten natürlichen Proteinhydrolysate, also der Hydrolysate natürlicher Proteine, lässt sich in Proteinhydrolysate pflanzlichen und tierischen Ursprungs unterteilen.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden u.a. unter den Handelsnamen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Pflanzliche Proteinhydrolysate sind beispielsweise Soja-, Mandel-, Erbsen-, Kartoffel- und Weizenproteinhydrolysate. Solche Produkte sind u.a. unter den Handelsnamen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Die Gruppe der marinen Proteinhydrolysate umfasst Proteinhydrolysate tierischen und pflanzlichen Ursprunges. Zu den marinen Proteinhydrolysaten zählen beispielsweise Kollagenhydrolysate von Fischen oder Algen, Proteinhydrolysate von Muscheln und die Perlenhydrolysate. Beispiele für erfindungsgemäße Perlenhydrolysate oder Perlenextrakte sind die unter den Handelsnamen Pearl Protein Extract BG® oder Crodarom® Pearl vertriebenen Substanzgemische.

Besonders bevorzugt sind Proteinhydrolysate auf der Basis von Kollagen, Seidenprotein, Keratin, Milchprotein, Sojaprotein, Mandelprotein, Weizenprotein oder Perlen. Höchst bevorzugt ist der Einsatz der Hydrolysate von Keratin, Seidenprotein und Weizenprotein.

Bevorzugte kosmetische Zubereitungen a) enthalten bezogen auf ihr Gesamtgewicht 0,01 bis 10 Gew.-%, vorzugsweise 0,05 bis 5,0 Gew.-% und insbesondere 0,1 bis 3,0 Gew.-% Proteinhydrolysat.

Die letzte Gruppe bevorzugter Pflegekomponenten a2) bilden die Wachse. Das Wachs a2) kann natürlichen oder synthetischen Ursprungs sein. Bevorzugte Wachse schmelzen oberhalb von 40°C, besonders bevorzugt oberhalb von 50°C, insbesondere bei Temperaturen zwischen 50°C und 90°C.

Als Wachse a2) können erfindungsgemäß feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2-6 C-Atomen, Fettsäuremonoalkanolamide mit einem C12-22-Acylrest und einem C2-4-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen.

Die Wachskomponenten können auch aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen ausgewählt werden, sofern die Wachskomponente oder die Gesamtheit der Wachskomponenten bei Raumtemperatur fest sind. Die Wachskomponenten können beispielsweise aus der Gruppe der C16-36-Alkylstearate, der C10-40-Alkylstearate, der C2-40-Alkylisostearate, der C20-40-Dialkylester von Dimersäuren, der C18-38-Alkylhydroxystearoylstearate, der C20-40-Alkylerucate ausgewählt werden, ferner sind C30-50-Alkylbienenwachs sowie Cetearylbehenat einsetzbar. Auch Silikonwachse, zum Beispiel Stearyltrimethylsilan/Stearylalkohol sind gegebenenfalls vorteilhaft. Bevorzugte Wachskomponenten sind die Ester aus gesättigten, einwertigen C20-C60-Alkoholen und gesättigten C8-C30-Monocarbonsäuren, insbesondere ein C20-C40-Alkylstearat bevorzugt, das unter dem Namen Kesterwachs® K82H von der Firma Koster Keunen Inc. erhältlich ist. Das Wachs oder die Wachskomponenten sollten bei 25 °C fest sein, jedoch im Bereich von 35-95 °C schmelzen, wobei ein Bereich von 45-85 ° C bevorzugt ist.

Weitere bevorzugte Wachskomponenten sind Fettalkohole. Als Fettalkohole können beispielsweise Stearylalkohol, Cetylalkohol, Laurylalkohol, Myristylalkohol, Arachidylalkohol, Caprylalkohol, Caprinalkohol und Behenylalkohol eingesetzt werden.

Bevorzugt ist das Wachs a2) aus Bienenwachs (Cera Alba), Carnaubawachs, Candelillawachs, Montanwachs, Cetylpalmitat, mikrokristallinen Wachsen (mikrokristalline Paraffine) und Gemischen davon ausgewählt. Besonders bevorzugt ist der Einsatz einer Pflegekomponente a2) aus der Gruppe Bienenwachs (Cera Alba), Carnaubawachs und mikrokristallinen Wachsen (mikrokristalline Paraffine).

Natürliche, chemisch modifizierte und synthetische Wachse können alleine oder in Kombination eingesetzt werden. Die erfindungsgemäße Lehre umfasst somit auch den kombinierten Einsatz von mehreren Wachsen. Weiterhin sind auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax® GP 200 (eine Mischung von Stearylalkohol und Polyethylenglykolstearat mit einem Schmelzpunkt von 47-51 deg. C; Hersteller: Croda) und "Weichceresin® FL 400" (ein Vaseline/Vaselinöl/Wachs-Gemisch mit einem Schmelzpunkt von 50-54 deg. C; Hersteller: Parafluid Mineralölgesellschaft) sind Beispiele für erfindungsgemäß bevorzugt eingesetzte Mischungen. Eine weitere besonders bevorzugte Mischung von Wachsen a2) umfasst Bienenwachs und Carnaubawachs, gegebenenfalls in Kombination mit mikrokristallinem Wachs.

Bevorzugte kosmetische Zubereitungen a) enthalten bezogen auf ihr Gesamtgewicht 0,01 bis 10 Gew.-%, vorzugsweise 0,05 bis 5,0 Gew.-% und insbesondere 0,1 bis 3,0 Gew.-% Wachs.

Die offenbarten kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck po, zum Beispiel den umgebenden Luftdruck (p₀ = 1,000 bar), entlastet werden kann, wodurch mindestens teilweise eine Verdampfung der kosmetischen Zubereitung a) erreicht wird.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung wird die kosmetische Zubereitung a) einer Düse zugeführt, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher zwingend notwendig. Der spezifische Düsentyp oder die spezifische Düsengestaltung wird in Abhängigkeit von den jeweiligen Sprühnebeleigenschaften gezielt festgelegt.

Zusammenfassend verfügt eine bevorzugte Vorrichtung zur Entspannungsverdampfung über
b1) einen mittels eines Ventils zu verschließenden und zu öffnenden Behälter b1), welcher den geschlossenen Innenraum definiert, in dem die kosmetische Zubereitung aufgenommen werden kann,
b2) eine Heizvorrichtung b2), welche es ermöglicht eine in dem Behälter b1) befindliche kosmetische Zubereitung zu erhitzen. und eine
Düse b3), welche eine Zerstäubung der aus dem Behälter entweichenden kosmetischen Zubereitung a) ermöglicht. Alternativ zu einem Ventil kann auch ein vergleichbar wirkendes Schließelement, welches eine zugehörige Öffnung im Behälter durch entsprechende Positionsänderung verschließen oder freigeben kann, zum Einsatz kommen

Weiters offenbart ist ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann.

Weiters offenbart ist daher ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht ein in dem Behälter b1) befindliche kosmetische Zubereitung zu erhitzen
   b3) eine Düse b3), welche eine Zerstäubung der kosmetischen Zubereitung a) ermöglicht.

Weiters offenbart ist mit anderen Worten ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann,
   - die erhitzte kosmetische Zubereitung a) aus dem Innenraum des Behälters b1) unter Druckminderung in die Umgebung entspannt werden kann.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Weiters offenbart ist daher ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Weiters offenbart ist daher ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht.

Weiters offenbart ist daher ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst.

Offenbart werden weiterhin kosmetische Produkte, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Offenbart wird auch ein kosmetisches Produkt, umfassend
a) eine kosmetische Zubereitung, enthaltend bezogen auf ihr Gesamtgewicht
   a1) 70 bis 99 Gew.-% polares Lösungsmittel;
   a2) mindestens 0,1 Gew.-% Pflegekomponente;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den zuvor beschriebenen Inhaltsstoffen a1) und a2) können die erfindungsgemäßen kosmetischen Mittel weitere Wirk-, Hilfs- und Pflegestoffe enthalten.

In einer bevorzugten Ausführungsform enthält die kosmetische Zubereitung a) weiterhin ein nichtionisches Tensid a5), vorzugsweise ein nichtionisches Tensid aus der Gruppe der PEG-Derivate von hydriertem Ricinusöl, besonders bevorzugt PEG-40 Hydrogenated Castor Oil umfasst. Besonders bevorzugte nichtionische Tenside sind PEG-Derivate von hydriertem Ricinusöl, die z. B. unter der Bezeichnung PEG Hydrogenated Castor Oil erhältlich sind, wie beispielsweise PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß besonders bevorzugt ist die Verwendung von PEG-40 Hydrogenated Castor Oil. Der Gewichtsanteil des nichtionischen Tensid, insbesondere von PEG-40 Hydrogenated Castor Oil am Gesamtgewicht der kosmetischen Zubereitung a) beträgt vorzugsweise 0,05 bis 2,0 Gew.-%, bevorzugt 0,1 bis 1,5 Gew.-% und insbesondere 0,2 bis 1,0 Gew.-%.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| kationisches Tensid | 0,10 bis 10 | 0,05 bis 5,0 | 0,1 bis 3,0 | 0,5 | 0,2 |
| optionale Additive | add 100 | add 100 | add 100 | add 100 | add 100 |

| | Formel 6 | Formel 7 | Formel 8 | Formel 9 | Formel 10 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| Silikon | 0,10 bis 30 | 0,05 bis 20 | 0,1 bis 10 | 0,3 | 3,3 |
| optionale Additive | add 100 | add 100 | add 100 | add 100 | add 100 |

| | Formel 11 | Formel 12 | Formel 13 | Formel 14 | Formel 15 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| Polyol | 0,10 bis 20 | 0,05 bis 10 | 0,1 bis 5,0 | 0,2 | 0,1 |
| optionale Additive | add 100 | add 100 | add 100 | add 100 | add 100 |

| | Formel 16 | Formel 17 | Formel 18 | Formel 19 | Formel 20 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 21 | Formel 22 | Formel 23 | Formel 24 | Formel 25 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 26 | Formel 27 | Formel 28 | Formel 29 | Formel 30 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Silikon | 0,10 bis 30 | 0,05 bis 20 | 0,1 bis 10 | 0,3 | 3,3 |
| optionale Additive | add 100 | add 100 | add 100 | add 100 | add 100 |

| | Formel 31 | Formel 32 | Formel 33 | Formel 34 | Formel 35 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Polyol | 0,10 bis 20 | 0,05 bis 10 | 0,1 bis 5,0 | 0,2 | 0,1 |
| optionale Additive | add 100 | add 100 | add 100 | add 100 | add 100 |

| | Formel 36 | Formel 37 | Formel 38 | Formel 39 | Formel 40 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 41 | Formel 42 | Formel 43 | Formel 44 | Formel 45 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 46 | Formel 47 | Formel 48 | Formel 49 | Formel 50 |
|---|---|---|---|---|---|
| polares Lösungsmittel a1) | 80 bis 99 | 90 bis 98 | 92 bis 97 | 95,8 | 94,4 |
| Silikon | 0,10 bis 30 | 0,05 bis 20 | 0,1 bis 10 | 0,3 | 3,3 |
| Polyol | 0,10 bis 20 | 0,05 bis 10 | 0,1 bis 5,0 | 0,2 | 0,1 |
| optionale Additive | add 100 | add 100 | add 100 | add 100 | add 100 |

| | Formel 51 | Formel 52 | Formel 53 | Formel 54 | Formel 55 |
|---|---|---|---|---|---|
| polares Lösungsmittel a1) | 80 bis 99 | 90 bis 98 | 92 bis 97 | 95,8 | 94,4 |
| Silikon | 0,10 bis 30 | 0,05 bis 20 | 0,1 bis 10 | 0,3 | 3,3 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 56 | Formel 57 | Formel 58 | Formel 59 | Formel 60 |
|---|---|---|---|---|---|
| polares Lösungsmittel a1) | 80 bis 99 | 90 bis 98 | 92 bis 97 | 95,8 | 94,4 |
| Silikon | 0,10 bis 30 | 0,05 bis 20 | 0,1 bis 10 | 0,3 | 3,3 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 61 | Formel 62 | Formel 63 | Formel 64 | Formel 65 |
|---|---|---|---|---|---|
| polares Lösungsmittel a1) | 80 bis 99 | 90 bis 98 | 92 bis 97 | 95,8 | 94,4 |
| Proteinhydrolysat | 0,10 bis 10 | 0,05 bis 5,0 | 0,1 bis 3,0 | 0,1 | 0,2 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 66 | Formel 67 | Formel 68 | Formel 69 | Formel 70 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| kationisches Tensid | 0,10 bis 10 | 0,05 bis 5,0 | 0,1 bis 3,0 | 0,5 | 0,2 |
| Silikon | 0,10 bis 30 | 0,05 bis 20 | 0,1 bis 10 | 0,3 | 3,3 |
| optionale Additive | add 100 | add 100 | add 100 | add 100 | add 100 |

| | Formel 71 | Formel 72 | Formel 73 | Formel 74 | Formel 75 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| kationisches Tensid | 0,10 bis 10 | 0,05 bis 5,0 | 0,1 bis 3,0 | 0,5 | 0,2 |
| Polyol | 0,10 bis 20 | 0,05 bis 10 | 0,1 bis 5,0 | 0,2 | 0,1 |
| optionale Additive | add 100 | add 100 | add 100 | add 100 | add 100 |

| | Formel 76 | Formel 77 | Formel 78 | Formel 79 | Formel 80 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| kationisches Tensid | 0,10 bis 10 | 0,05 bis 5,0 | 0,1 bis 3,0 | 0,5 | 0,2 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 81 | Formel 82 | Formel 83 | Formel 84 | Formel 85 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| kationisches Tensid | 0,10 bis 10 | 0,05 bis 5,0 | 0,1 bis 3,0 | 0,5 | 0,2 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 86 | Formel 87 | Formel 88 | Formel 89 | Formel 90 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Silikon | 0,10 bis 30 | 0,05 bis 20 | 0,1 bis 10 | 0,3 | 3,3 |
| Polyol | 0,10 bis 20 | 0,05 bis 10 | 0,1 bis 5,0 | 0,2 | 0,1 |
| optionale Additive | add 100 | add 100 | add 100 | add 100 | add 100 |

| | Formel 91 | Formel 92 | Formel 93 | Formel 94 | Formel 95 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Silikon | 0,10 bis 30 | 0,05 bis 20 | 0,1 bis 10 | 0,3 | 3,3 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 96 | Formel 97 | Formel 98 | Formel 99 | Formel 100 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Silikon | 0,10 bis 30 | 0,05 bis 20 | 0,1 bis 10 | 0,3 | 3,3 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 101 | Formel 102 | Formel 103 | Formel 104 | Formel 105 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Polyol | 0,10 bis 20 | 0,05 bis 10 | 0,1 bis 5,0 | 0,2 | 0,1 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 106 | Formel 107 | Formel 108 | Formel 109 | Formel 110 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Polyol | 0,10 bis 20 | 0,05 bis 10 | 0,1 bis 5,0 | 0,2 | 0,1 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 111 | Formel 112 | Formel 113 | Formel 114 | Formel 115 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| Proteinhydrolysat | 0,10 bis 10 | 0,05 bis 5,0 | 0,1 bis 3,0 | 0,1 | 0,2 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 116 | Formel 117 | Formel 118 | Formel 119 | Formel 120 |
|---|---|---|---|---|---|
| polares Lösungsmittel a1) | 80 bis 99 | 90 bis 98 | 92 bis 97 | 95,8 | 94,4 |
| Silikon | 0,10 bis 30 | 0,05 bis 20 | 0,1 bis 10 | 0,3 | 3,3 |
| Polyol | 0,10 bis 20 | 0,05 bis 10 | 0,1 bis 5,0 | 0,2 | 0,1 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 121 | Formel 122 | Formel 123 | Formel 124 | Formel 125 |
|---|---|---|---|---|---|
| polares Lösungsmittel a1) | 80 bis 99 | 90 bis 98 | 92 bis 97 | 95,8 | 94,4 |
| Silikon | 0,10 bis 30 | 0,05 bis 20 | 0,1 bis 10 | 0,3 | 3,3 |
| Polyol | 0,10 bis 20 | 0,05 bis 10 | 0,1 bis 5,0 | 0,2 | 0,1 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 126 | Formel 127 | Formel 128 | Formel 129 | Formel 130 |
|---|---|---|---|---|---|
| polares Lösungsmittel a1) | 80 bis 99 | 90 bis 98 | 92 bis 97 | 95,8 | 94,4 |
| Silikon | 0,10 bis 30 | 0,05 bis 20 | 0,1 bis 10 | 0,3 | 3,3 |
| Proteinhydrolysat | 0,10 bis 10 | 0,05 bis 5,0 | 0,1 bis 3,0 | 0,1 | 0,2 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 131 | Formel 132 | Formel 133 | Formel 134 | Formel 135 |
|---|---|---|---|---|---|
| polares Lösungsmittel a1) | 80 bis 99 | 90 bis 98 | 92 bis 97 | 95,8 | 94,4 |
| Polyol | 0,10 bis 20 | 0,05 bis 10 | 0,1 bis 5,0 | 0,2 | 0,1 |
| Proteinhydrolysat | 0,10 bis 10 | 0,05 bis 5,0 | 0,1 bis 3,0 | 0,1 | 0,2 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 136 | Formel 137 | Formel 138 | Formel 139 | Formel 140 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| kationisches Tensid | 0,10 bis 10 | 0,05 bis 5,0 | 0,1 bis 3,0 | 0,5 | 0,2 |
| Silikon | 0,10 bis 30 | 0,05 bis 20 | 0,1 bis 10 | 0,3 | 2,3 |
| Polyol | 0,10 bis 20 | 0,05 bis 10 | 0,1 bis 5,0 | 0,2 | 0,1 |
| Proteinhydrolysat | 0,10 bis 10 | 0,05 bis 5,0 | 0,1 bis 3,0 | 0,1 | 0,2 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

| | Formel 141 | Formel 142 | Formel 143 | Formel 144 | Formel 145 |
|---|---|---|---|---|---|
| polares Lösungsmittel a1) | 80 bis 99 | 90 bis 98 | 92 bis 97 | 95,8 | 94,4 |
| kationisches Polymer | 0,10 bis 10 | 0,05 bis 5,0 | 0,1 bis 3,0 | 0,3 | 0,8 |
| optionale Additive | add 100 | add 100 | add 100 | add 100 | add 100 |

| | Formel 146 | Formel 147 | Formel 148 | Formel 149 | Formel 150 |
|---|---|---|---|---|---|
| polares Lösungsmittel a1) | 80 bis 99 | 90 bis 98 | 92 bis 97 | 95,8 | 94,4 |
| kationisches Tensid | 0,10 bis 10 | 0,05 bis 5,0 | 0,1 bis 3,0 | 0,5 | 0,2 |
| optionale Additive | add 100 | add 100 | add 100 | add 100 | add 100 |

| | Formel 151 | Formel 152 | Formel 153 | Formel 154 | Formel 155 |
|---|---|---|---|---|---|
| polares Lösungsmittel a1) | 80 bis 99 | 90 bis 98 | 92 bis 97 | 95,8 | 94,4 |
| Silikon | 0,10 bis 30 | 0,05 bis 20 | 0,1 bis 10 | 0,3 | 2,3 |
| optionale Additive | add 100 | add 100 | add 100 | add 100 | add 100 |

| | Formel 156 | Formel 157 | Formel 158 | Formel 159 | Formel 160 |
|---|---|---|---|---|---|
| polares Lösungsmittel a1) | 80 bis 99 | 90 bis 98 | 92 bis 97 | 95,8 | 94,4 |
| Polyol | 0,10 bis 20 | 0,05 bis 10 | 0,1 bis 5,0 | 0,2 | 0,1 |
| optionale Additive | add 100 | add 100 | add 100 | add 100 | add 100 |

| | Formel 161 | Formel 162 | Formel 163 | Formel 164 | Formel 165 |
|---|---|---|---|---|---|
| polares Lösungsmittel a1) | 80 bis 99 | 90 bis 98 | 92 bis 97 | 95,8 | 94,4 |
| Proteinhydrolysat | 0,10 bis 10 | 0,05 bis 5,0 | 0,1 bis 3,0 | 0,1 | 0,2 |
| optionale Additive | add 100 | add 100 | add 100 | add 100 | add 100 |

| | Formel 166 | Formel 167 | Formel 168 | Formel 169 | Formel 170 |
|---|---|---|---|---|---|
| polares Lösungsmittel a1) | 80 bis 99 | 90 bis 98 | 92 bis 97 | 95,8 | 94,4 |
| Wachs | 0,10 bis 10 | 0,05 bis 5,0 | 0,1 bis 3,0 | 1,0 | 1,3 |
| optionale Additive | add 100 | add 100 | add 100 | add 100 | add 100 |

Ganz besonders bevorzugte kosmetische Zubereitungen enthalten neben den zuvor beschriebenen Bestandteilen a1) und a2) nur geringe Mengen weiterer Wirk- und Hilfsstoffe. Kosmetische Zubereitungen, dadurch gekennzeichnet, dass der Gewichtsanteil der Bestandteile a1), a2) am Gesamtgewicht der kosmetischen Zubereitung mindestens 86 Gew.-%, vorzugsweise mindestens 90 Gew.-% und insbesondere mindestens 94 Gew.-% beträgt, sind aufgrund ihrer einfachen Herstellbarkeit und guten kosmetischen Wirkung besonders bevorzugt..

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 70 bis 99 Gew.-% polares Lösungsmittel;
a2) mindestens 0,1 Gew.-% Pflegekomponente;
als Prozessgut in einer Vorrichtung zur Entspannungsverdampfung.

Gegenstand der vorliegenden Anmeldung ist zudem die Verwendung eines erfindungsgemäßen Produktes zur Beaufschlagung keratinhaltiger Fasern, insbesondere menschlicher Haare, mit einer kosmetischen Zubereitung a) bzw. zur Pflege keratinhaltiger Fasern, insbesondere menschlicher Haare.

Ein Verfahren zur temporären Verformung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 70 bis 99 Gew.-% polares Lösungsmittel;
a2) mindestens 0,1 Gew.-% Pflegekomponente;
beaufschlagt werden, ist ein weiterer Gegenstand der vorliegenden Anmeldung. Die kosmetische Zubereitung a) wird mittels der Vorrichtung zur Entspannungsverdampfung in einen Sprühnebel überführt, welcher nachfolgend die keratinhaltigen Fasern beaufschlagt.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Offenbart wird auch ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 70 bis 99 Gew.-% polares Lösungsmittel;
a2) mindestens 0,1 Gew.-% Pflegekomponente;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

## Patentansprüche

1. Kosmetisches Produkt, umfassend
a) eine flüssige kosmetische Zubereitung, enthaltend bezogen auf ihr Gesamtgewicht
a1) 70 bis 99 Gew.-% polares Lösungsmittel;
a2) mindestens 0,1 Gew.-% Pflegekomponente;
b) eine Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung auf Temperaturen oberhalb der Umgebungstemperatur in einer Weise zu erhitzen, dass in dem Behälter ein Druck oberhalb des Umgebungsdrucks entsteht, sowie die erhitzte und unter erhöhtem Druck stehende kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

2. Kosmetisches Produkt nach Anspruch 1, **dadurch gekennzeichnet, dass** der Gewichtsanteil des polaren Lösungsmittels a1) am Gesamtgewicht der kosmetischen Zubereitung a) 80 bis 99 Gew.-%, vorzugsweise 90 bis 98 Gew.-% und insbesondere 92 bis 97 Gew.-% beträgt.

3. Kosmetisches Produkte nach einem der vorherigen Ansprüche, **dadurch gekennzeichnet, dass** das polare Lösungsmittel a1) ausgewählt ist aus der Gruppe Wasser und Ethanol.

4. Kosmetisches Produkt nach einem der vorherigen Ansprüche, **dadurch gekennzeichnet, dass** der Gewichtsanteil der Pflegekomponente a2) am Gesamtgewicht der kosmetischen Zubereitung a) 0,2 bis 18 Gew.-%, vorzugsweise 0,5 bis 12 Gew.-% und insbesondere 1,0 bis 10 Gew.-% beträgt.

5. Kosmetisches Produkt nach einem der vorherigen Ansprüche, **dadurch gekennzeichnet, dass** die Pflegekomponte a2) ausgewählt ist aus der Gruppe der
i) kationischen Pflegekomponenten, insbesondere aus der Gruppe der kationischen Polymere und kationischen Tenside;
ii) Silikone, insbesondere aus der Gruppe der
- alkoxylierten Dimethicone, vorzugsweise aus der Gruppe der ethoxylierten/propoxylierten Dimethicone;
- aminofunktionellen Silikone;
iii) Polyole;
iv) Proteinhydrolysate, insbesondere aus der Gruppe der Gruppe der Hydrolysate von Keratin, Seidenprotein und Weizenprotein.

6. Kosmetisches Produkt nach einem der vorherigen Ansprüche **dadurch gekennzeichnet, dass** die kosmetische Zubereitung mindestens zwei, vorzugsweise mindestens drei und insbesondere mindestens vier voneinander verschiedene Pflegekomponenten a2) umfasst.

7. Verwendung einer flüssigen kosmetischen Zubereitung a) enthaltend, bezogen auf ihr Gesamtgewicht,
a1) 70 bis 99 Gew.-% polares Lösungsmittel;
a2) mindestens 0,1 Gew.-% Pflegekomponente;
als Prozessgut in einer Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung auf Temperaturen oberhalb der Umgebungstemperatur in einer Weise zu erhitzen, dass in dem Behälter ein Druck oberhalb des Umgebungsdrucks entsteht, sowie die erhitzte und unter erhöhtem Druck stehende kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

8. Verwendung eines Produkts nach einem der Ansprüche 1 bis 6 zur Beaufschlagung keratinhaltiger Fasern, insbesondere menschlicher Haare, mit einer kosmetischen Zubereitung a).

9. Verwendung eines Produkts nach einem der Ansprüche 1 bis 6 zur Pflege keratinhaltiger Fasern, insbesondere menschlicher Haare.

10. Verfahren zur Pflege keratinhaltiger Fasern, insbesondere menschlicher Haare, bei welchem die keratinhaltigen Fasern mittels einer Vorrichtung zur Entspannungsverdampfung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung auf Temperaturen oberhalb der Umgebungstemperatur in einer Weise zu erhitzen, dass in dem Behälter ein Druck oberhalb des Umgebungsdrucks entsteht, sowie die erhitzte und unter erhöhtem Druck stehende kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
mit einer flüssigen kosmetischen Zubereitung a) enthaltend bezogen auf ihr Gesamtgewicht
a1) 70 bis 99 Gew.-% polares Lösungsmittel;
a2) mindestens 0,1 Gew.-% Pflegekomponente;
beaufschlagt werden.

11. Verfahren nach Anspruch 10, **dadurch gekennzeichnet, dass**
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter überführt wird;
- nachfolgend der Zugang zwischen Vorratsbehälter und Behälter durch ein Bauteil zur Durchflussregelung, mittels dessen der Durchfluss der kosmetischen Zubereitung a) aus dem Vorratsbehälter in den Behälter unterbrochen werden kann, unterbrochen wird;
- nachfolgend die in dem gegen die Umgebung abgeschlossenen Behälter befindliche kosmetische Zubereitung a) mittels einer Heizvorrichtung erhitzt wird, so dass der Druck im Inneren des Behälters auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter in einer Weise geöffnet wird, um mindestens eine Teilmenge, vorzugsweise mindestens 50 Gew.- %, bevorzugt mindestens 80 Gew.-% und insbesondere mindestens 90 Gew.-% der in dem Behälter befindlichen kosmetischen Zubereitung aus dem Behälter in die Umgebung abzugeben sowie unter Minderung des zum Zeitpunkt der Behälteröffnung in dem Behälter herrschenden Drucks zu entspannen.

## Claims

1. A cosmetic product comprising:
a) a liquid cosmetic preparation containing, based on the total weight thereof,
a1) 70 to 99 wt.% of polar solvent;
a2) at least 0.1 wt.% care component;
b) a device for flash evaporation of the cosmetic preparation a), comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating the cosmetic preparation a) located in the closed interior of the container under increased pressure to temperatures above the ambient temperature in such a way that a pressure above the ambient pressure is produced in the container, and for releasing, under reduced pressure, the heated cosmetic preparation a) that is under increased pressure from the interior of the container into the surroundings,
b4) a nozzle which allows atomization of the cosmetic preparation a) escaping from the container.

2. The cosmetic product according to claim 1, **characterized in that** the proportion by weight of the polar solvent a1) with respect to the total weight of the cosmetic preparation a) is 80 to 99 wt.%, preferably 90 to 98 wt.% and in particular 92 to 97 wt.%.

3. The cosmetic product according to one of the preceding claims, **characterized in that** the polar solvent a1) is selected from the group water and ethanol.

4. The cosmetic product according to one of the preceding claims, **characterized in that** the proportion by weight of the care component a2) with respect to the total weight of the cosmetic preparation a) is 0.2 to 18 wt.%, preferably 0.5 to 12 wt.% and in particular 1.0 to 10 wt.%.

5. The cosmetic product according to one of the preceding claims, **characterized in that** the care component a2) is selected from the group of
i) cationic care components, in particular from the group of cationic polymers and cationic surfactants;
ii) silicones, in particular from the group of
- alkoxylated dimethicones, preferably from the group of ethoxylated/propoxylated dimethicones;
- amino-functional silicones;
iii) polyols;
iv) protein hydrolyzates, in particular from the group of the group of hydrolyzates of keratin, silk protein and wheat protein.

6. The cosmetic product according to one of the preceding claims, **characterized in that** the cosmetic preparation comprises at least two, preferably at least three and in particular at least four different care components a2).

7. The use of a liquid cosmetic preparation a) containing, based on the total weight thereof,
a1) 70 to 99 wt.% of polar solvent;
a2) at least 0.1 wt.% care component;
as a process material in a device for flash evaporation of the cosmetic preparation a), comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating the cosmetic preparation a) located in the closed interior of the container under increased pressure to temperatures above the ambient temperature in such a way that a pressure above the ambient pressure is produced in the container, and for releasing, under reduced pressure, the heated cosmetic preparation a) that is under increased pressure from the interior of the container into the surroundings,
b4) a nozzle which allows atomization of the cosmetic preparation a) escaping from the container.

8. The use of a product according to one of claims 1 to 6 for applying a cosmetic preparation a) to keratin-containing fibers, in particular human hair.

9. The use of a product according to one of claims 1 to 6 for the care of keratin-containing fibers, in particular human hair.

10. A method for the care of keratin-containing fibers, in particular human hair, in which, by means of a device for flash evaporation comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating the cosmetic preparation a) located in the closed interior of the container under increased pressure to temperatures above the ambient temperature in such a way that a pressure above the ambient pressure is produced in the container, and for releasing, under reduced pressure, the heated cosmetic preparation a) that is under increased pressure from the interior of the container into the surroundings,
b4) a nozzle which allows atomization of the cosmetic preparation a) escaping from the container,
a liquid cosmetic preparation a) containing, based on the total weight thereof,
a1) 70 to 99 wt.% of polar solvent;
a2) at least 0.1 wt.% care component;
can be applied to the keratin-containing fibers.

11. The method according to claim 10, **characterized in that**
- some of the cosmetic preparation a) located in a reservoir, in the interior of which a pressure prevails that corresponds to the ambient pressure, is transferred from said reservoir into a container;
- the access between the reservoir and the container is subsequently interrupted by a component for controlling flow, by means of which the flow of the cosmetic preparation a) from the reservoir into the container can be interrupted;
- the cosmetic preparation a) located in the container that is sealed against the surroundings is subsequently heated by means of a heating device such that the pressure inside the container increases to values above the ambient pressure, preferably to values between 1.1 and 8 bar, in particular to values between 1.2 and 4 bar;
- the container that is pressurized above the ambient pressure is subsequently opened in such a way as to discharge at least some, preferably at least 50 wt.%, more preferably at least 80 wt.% and in particular at least 90 wt.%, of the cosmetic preparation located in the container from the container into the surroundings and to release said preparation with reduction of the pressure prevailing in the container at the time the container is opened.

## Revendications

1. Produit cosmétique, comprenant
a) une préparation cosmétique liquide contenant, par rapport à son poids total
a1) 70 à 99 % en poids de solvant polaire ;
a2) au moins 0,1 % en poids de composant de soin ;
b) un dispositif de vaporisation par détente de la préparation cosmétique a) comportant
b1) un récipient définissant un espace intérieur fermé dans lequel la préparation cosmétique peut être reçue,
b2) une soupape ou un élément de fermeture à action comparable permettant de fermer et d'ouvrir l'espace interne du récipient rempli au moins partiellement de préparation cosmétique a),
b3) un dispositif de chauffage destiné à chauffer la préparation cosmétique a) se trouvant dans l'espace intérieur fermé du récipient à des températures supérieures à la température ambiante tout en augmentant la pression, de sorte qu'une pression supérieure à la pression ambiante soit produite dans le récipient, et à détendre la préparation cosmétique a) chauffée et pressurisée hors de l'espace intérieur du récipient tout en réduisant la pression dans l'environnement,
b4) une buse permettant d'atomiser la préparation cosmétique a) s'échappant du récipient.

2. Produit cosmétique selon la revendication 1, **caractérisé en ce que** la proportion en poids du solvant polaire a1) par rapport au poids total de la préparation cosmétique a) est comprise entre 80 et 99 % en poids, de préférence entre 90 et 98 % en poids et en particulier entre 92 et 97 % en poids.

3. Produit cosmétique selon l'une des revendications précédentes, **caractérisé en ce que** le solvant polaire a1) est choisi dans le groupe constitué de l'eau et de l'éthanol.

4. Produit cosmétique selon l'une des revendications précédentes, **caractérisé en ce que** la proportion en poids du composant de soins a2) par rapport au poids total de la préparation cosmétique a) est comprise entre de 0,2 et 18 % en poids, de préférence entre 0,5 et 12 % en poids et en particulier entre 1,0 et 10 % en poids.

5. Produit cosmétique selon l'une des revendications précédentes, **caractérisé en ce que** le composant de soins a2) est choisi dans le groupe constitué des
i) composants de soins cationiques, en particulier dans le groupe constitué des polymères cationiques et des tensioactifs cationiques ;
ii) des silicones, en particulier dans le groupe constitué des
- diméthicones alcoxylées, de préférence dans le groupe constitué des diméthicones éthoxylées/propoxylées ;
- des silicones aminofonctionnels ;
iii) des polyols ;
iv) des hydrolysats de protéines, en particulier dans le groupe constitué des hydrolysats de kératine, des protéines de soie et des protéines de blé.

6. Produit cosmétique selon l'une des revendications précédentes, **caractérisé en ce que** la préparation cosmétique comprend au moins deux, de préférence au moins trois et en particulier au moins quatre composants de soins a2) différents.

7. Utilisation d'une préparation cosmétique a) liquide contenant, par rapport à son poids total,
a1) 70 à 99 % en poids de solvant polaire ;
a2) au moins 0,1 % en poids de composant de soin ;
comme produit de traitement dans un dispositif de vaporisation par détente de la préparation cosmétique a) comportant
b1) un récipient définissant un espace intérieur fermé dans lequel la préparation cosmétique peut être reçue,
b2) une soupape ou un élément de fermeture à action comparable permettant de fermer et d'ouvrir l'espace interne du récipient rempli au moins partiellement de préparation cosmétique a),
b3) un dispositif de chauffage destiné à chauffer la préparation cosmétique a) se trouvant dans l'espace intérieur fermé du récipient à des températures supérieures à la température ambiante tout en augmentant la pression, de sorte qu'une pression supérieure à la pression ambiante soit produite dans le récipient, et à détendre la préparation cosmétique a) chauffée et pressurisée hors de l'espace intérieur du récipient tout en réduisant la pression dans l'environnement,
b4) une buse permettant d'atomiser la préparation cosmétique a) s'échappant du récipient.

8. Utilisation d'un produit selon l'une des revendications 1 à 6 pour l'application d'une préparation cosmétique a) sur des fibres kératiniques, en particulier des cheveux humains.

9. Utilisation d'un produit selon l'une des revendications 1 à 6 pour le soin de fibres kératiniques, en particulier de cheveux humains.

10. Procédé de soin de fibres kératiniques, en particulier de cheveux humains, dans lequel on applique sur les fibres kératiniques, au moyen d'un dispositif de vaporisation par détente comportant
b1) un récipient définissant un espace intérieur fermé dans lequel la préparation cosmétique peut être reçue,
b2) une soupape ou un élément de fermeture à action comparable permettant de fermer et d'ouvrir l'espace interne du récipient rempli au moins partiellement de préparation cosmétique a),
b3) un dispositif de chauffage destiné à chauffer la préparation cosmétique a) se trouvant dans l'espace intérieur fermé du récipient à des températures supérieures à la température ambiante tout en augmentant la pression, de sorte qu'une pression supérieure à la pression ambiante soit produite dans le récipient, et à détendre la préparation cosmétique a) chauffée et pressurisée hors de l'espace intérieur du récipient tout en réduisant la pression dans l'environnement,
b4) une buse permettant d'atomiser la préparation cosmétique a) s'échappant du récipient,
une préparation cosmétique a) liquide contenant, par rapport à son poids total
a1) 70 à 99 % en poids de solvant polaire ;
a2) au moins 0,1 % en poids de composant de soin ;

11. Procédé selon la revendication 10, **caractérisé en ce que**,
- à partir d'un réservoir de stockage à l'intérieur duquel règne une pression égale à la pression ambiante, une fraction de la préparation cosmétique a) présente dans ce réservoir de stockage est transférée dans un récipient ;
- on interrompt ensuite l'accès entre le réservoir de stockage et le récipient par un élément de régulation de flux au moyen duquel le flux de la préparation cosmétique a) depuis le réservoir de stockage jusqu'au récipient peut être interrompu ;
- on chauffe ensuite la préparation cosmétique a) présente dans le récipient isolé de l'environnement au moyen d'un dispositif de chauffage, de sorte que la pression à l'intérieur du récipient dépasse la pression ambiante et atteint de préférence des valeurs comprises entre 1,1 et 8 bar, en particulier entre 1,2 et 4 bars ;
- le récipient, se trouvant à une pression supérieure à la pression ambiante, est ensuite ouvert de manière à libérer dans l'environnement au moins une fraction, de préférence au moins 50 % en poids, de manière préférée au moins 80 % en poids et en particulier au moins 90 % en poids de la préparation cosmétique présente dans le récipient et à provoquer sa sortie dans l'atmosphère tout en réduisant la pression régnant dans le récipient au moment de l'ouverture de celui-ci.
